# EUROPÄISCHE PATENTSCHRIFT

(11) **EP 0 673 912 B1**
(45) Veröffentlichungstag und Bekanntmachung des Hinweises auf die Patenterteilung: **23.12.1998**
(21) Anmeldenummer: 95103328.1
(22) Anmeldetag: 08.03.1995
(51) Int. Cl.: C07C 51/58, C07C 17/20, C07C 63/70, C07C 25/13

(54) **Verfahren zur Herstellung von 2-Halogen-4,5-difluor-benzofluoriden**
Process for preparing 2-halogeno-4,5-difluoro-benzofluorides
Procédé pour la préparation de 2-halogéno-4,5-difluoro-benzofluorides

(30) Priorität: 21.03.1994 DE 4409578
(43) Veröffentlichungstag der Anmeldung: 27.09.1995
(73) Patentinhaber: BAYER AG, 51368 Leverkusen (DE)
(72) Erfinder: Antons, Stefan, Dr., D-51373 Leverkusen (DE); Marhold, Albrecht, Dr., D-51373 Leverkusen (DE); Beitzke, Bernhard, Dr., D-51503 Rösrath (DE)

(56) Entgegenhaltungen:
- EP-A- 0 266 512
- DE-A- 3 420 796

## Beschreibung

Die vorliegende Erfindung betrifft ein besonders vorteilhaftes Verfahren zur Herstellung von Gemischen, die 2,4,5-Trifluor- und 2-Chlor-4,5-difluor-benzoylfluorid und weniger als 1 Gew.-% 4-Chlor-2,5-difluor-benzoylfluorid enthalten aus 2,4-Dichlor-5-fluorbenzoylchlorid.

2-Chlor-4,5-difluor-benzoylchlorid, die entsprechende Benzoesäure und der entsprechende Benzylalkohol sind wertvolle Zwischenprodukte zur Herstellung von 7-(substituierten) Piperazinyl-1-substituierten-6-fluoro-1,4-dihydro-4-oxo-3-chinolincarbonsäuren, die antibakterielle Wirkstoffe sind (siehe z.B. US-A 4 833 270). Dabei sind offenbar die Fluoratome in 4- und 5-Stellung von besonderer Bedeutung, denn diese sind im antibakteriellen Wirkstoff unverändert vorhanden, während das Fluoratom in 2-Stellung auf dem Weg zum antibakteriellen Wirkstoff abgespalten wird und deshalb an dieser Stelle im Zwischenprodukt sich auch ein Chloratom befinden kann (siehe auch EP-A 303 291).

Aus 2-(Fluor oder Chlor)-4,5-difluor-benzoylfluorid ist das entsprechende Benzoylchlorid, die entsprechende Benzoesäure und der entsprechende Benzylalkohol auf übliche Weise zugänglich.

Es ist bekannt, daß man 2,4,5-Trifluor-benzoylfluorid aus 2,4-Dichlor-5-fluorbenzoylfluorid durch Einwirkung von Kaliumfluorid in Gegenwart eines aprotischen Lösungsmittels herstellen kann (siehe DE-A 34 20 796). Die Ausbeute beträgt dabei 65,5 % d. Th. (siehe Beispiel 1 der DE-A). Ob und gegebenenfalls zu welchen Produkten die restlichen 34,5 % des Ausgangsmaterials umgesetzt werden ist nicht angegeben. Es besteht deshalb noch ein Bedürfnis nach einem Verfahren zur Herstellung von Zwischenprodukten für antibakterielle Wirkstoffe, bei denen diese Zwischenprodukte in höheren Ausbeuten erhalten werden.

Es wurde nun ein Verfahren zur Herstellung von Gemischen, die 2,4,5-Trifluor- und 2-Chlor-4,5-difluorbenzoylfluorid und weniger als 1 Gew.-% 4-Chlor-2,5-difluorbenzoylfluorid enthalten, gefunden, das dadurch gekennzeichnet ist, daß man 2,4-Dichlor-5-fluor-benzoylchlorid mit der 3,1- bis 4,5-fachen molaren Menge Kaliumfluorid in einem aprotischen Lösungsmittel umsetzt und das Reaktionsgemisch anschließend destilliert.

Das in das erfindungsgemäße Verfahren einsetzbare Ausgangsprodukt ist auf bekannte Weise aus 3-Fluortoluol zugänglich.

Als aprotische Lösungsmittel für das erfindungsgemäße Verfahren kommen z.B. Dimethylformamid, Dimethylsulfoxid, N-Methylpyrrolidon, Dimethylsulfon und Tetramethylensulfon (= Sulfolan) in Frage. Vorzugsweise wird Tetramethylensulfon eingesetzt. Das Lösungsmittel kann man beispielsweise in Mengen von 200 bis 1000 ml pro Mol Ausgangsmaterial der Formel (II) einsetzen.

Das erfindungsgemäße Verfahren kann z.B. bei Temperaturen im Bereich 150 bis 270°C durchgeführt werden. Bevorzugt sind Temperaturen im Bereich 180 bis 230°C.

Die Reaktionszeit kann beispielsweise zwischen 1 und 12 Stunden betragen.

Der Druck ist für das erfindungsgemäße Verfahren ohne besonderen Belang und kann praktisch beliebig gewählt werden. Im allgemeinen arbeitet man deshalb bei 0,5 bis 5 bar, insbesondere bei Normaldruck.

Die Aufarbeitung des Reaktionsgemisches erfolgt so, daß man es, vorzugsweise bei vermindertem Druck, destilliert. Man erhält dann Gemische von 2-Fluor- und 2-Chlor-4,5-difluorbenzoylfluoriden, die für die Weiterverarbeitung zu Arzneimitteln geeignet sind.

Bei einer bevorzugten Ausführungsform des erfindungsgemäßen Verfahrens geht man so vor, daß man Kaliumfluorid in Tetramethylensulfon vorlegt und bei vermindertem Druck eine kleine Menge Tetramethylensulfon abdestilliert, um damit gegebenenfalls verhandene kleine Mengen von Wasser zu entfernen. Dann kühlt man etwas ab, setzt 2,4-Dichlor-5-fluorbenzoylchlorid zu und erhitzt unter Rühren solange auf die gewünschte Reaktionstemperatur bis sich das Einsatzmaterial vollständig umgesetzt hat. Dann kühlt man wieder etwas ab und destilliert bei einem Druck im Bereich von 50 bis 200 mbar das erhaltene 2,4,5-Trifluor- und 2-Chlor-4,5-difluorbenzoylfluorid ab.

Das erfindungsgemäße Verfahren hat den überraschenden Vorteil, daß damit Zwischenprodukte für die Herstellung von antibakteriellen Wirkstoffen (Gemische von 2,4,5-Trifluor- und 2-Chlor-4,5-difluor-benzoylfluorid) in Ausbeuten von über 75 % d. Th. häufig über 85 % d. Th. erhalten werden können. Weiterhin ist überraschend, daß das für die Herstellung antibakterieller Wirkstoffe nicht brauchbare Isomere 4-Chlor-2,5-difluorbenzoylfluorid beim erfindungsgemäßen Verfahren entweder gar nicht oder nur in Spuren von deutlich unter 1 % gebildet wird.

Aus den erfindungsgemäß zugänglichen 2-Halogen-4,5-difluor-benzoylfluoriden lassen sich auf übliche und einfache Weise die entsprechenden Benzoylchloride, Benzoesäuren und Benzylalkohole erhalten, deren Einsatz zur Herstellung antibakterieller Wirkstoffe literaturbekannt ist. So kann man beispielsweise 2-Halogen-4,5-difluor-benzoylfluoride
a) zunächst mit Säure und anschließend mit Thionylchlorid umsetzen und so die entsprechenden Benzoylchloride erhalten oder
b) mit Siliziumtetrachlorid, gegebenenfalls in Gegenwart katalytischer Mengen Aluminiumtrichlorid oder mit Calciumchlorid umsetzen und so ebenfalls die entsprechenden Benzoylchloride erhalten oder
c) mit Natriumboranat reduzieren und so die entsprechenden Benzylalkohole erhalten oder
d) zunächst entsprechend a) oder b) zu Benzoylchloriden umsetzen und diese durch Verseifung in die entsprechende Benzoesäure überführen.

### Beispiele

### Beispiel 1

In einem 4-Halskolben mit Rührer, Thermometer, Stickstoff-Einleitung und Destillationsbrücke wurde eine Mischung aus 203,5 g Kaliumfluorid und 500 ml Sulfolan auf 200°C erwärmt. Durch Anlegen von Vakuum wurden bei einer Kopftemperatur von 170 bis 180°C 50 ml Sulfolan abdestilliert. Nach Abkühlung auf 150°C wurden 211 g 2,4-Dichlor-5-fluorbenzoylchlorid zugesetzt und 2 Stunden bei 200°C gerührt, bis sich das 2,4-Dichlor-5-fluorbenzoylchlorid vollständig umgesetzt hatte. Anschließend wurde das Reaktionsgemisch bei 100 mbar destilliert. Es wurden 171 g Destillat erhalten. Das Destillat bestand zu 50,5 Gew.-% aus 2,4,5-Trifluorbenzoylfluorid, zu 49,0 Gew.-% aus 2-Chlor-4,5-difluorbenzoylfluorid und enthielt nur Spuren des 4-Chlor-2,5-difluorbenzoylfluorids. Somit enthielt das Destillat über 99 Gew.-% der gewünschten Produkte.

### Beispiel 2

Zu einer Mischung von 500 ml Sulfolan und 232,4 g Kaliumfluorid wurden nach Abdestillieren (im Vakuum) von 50 ml Sulfolan bei 150°C 227,5 g 2,4-Dichlor-5-fluorbenzoylchlorid gegeben. Nach 2-stündiger Reaktion bei 200°C und Destillation des Gemisches wurden 179 g Produkt erhalten. Diese enthielt 52,7 Gew.-% Trifluorbenzoylfluorid, 46,7 Gew.-% 2-Chlor-4,5-difluor-benzoylfluorid, 0,3 Gew.-% 2,4-Dichlor-5-fluor-benzoylfluorid und 0,2 Gew.-% 4-Chlor-2,5-difluorbenzoylfluorid. Das Destillat enthielt somit 99,4 Gew.-% der erwünschten Produkte.

### Beispiele 3 bis 7

Es wurde verfahren wie in Beispiel 1, jedoch wurden die eingesetzten Mengen Kaliumfluorid und die Reaktionszeit variiert. Die erhaltenen Ergebnisse sind in der nachfolgenden Tabelle 1 zusammengestellt.

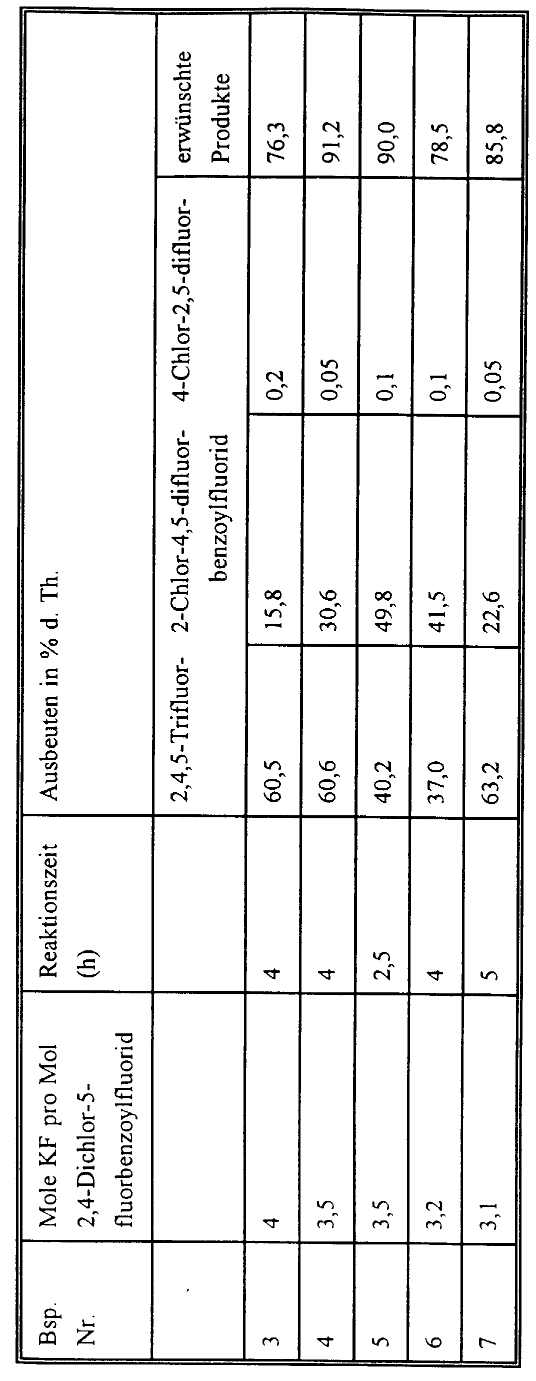

Beispiel 3 zeigt, daß eine Weiterführung der Fluorierung praktisch zu Verlusten in der Ausbeute erwünschter Produkte führt. Dieser Einfluß geht bei Senkung der Kaliumfluoridmenge deutlich zurück (siehe Beispiele 4 und 5). Beispiel 6 zeigt ebenfalls, daß auch mit wenig Kaliumfluorid gute Ergebnisse erzielt werden. Aus Beispiel 7 ist ersichtlich, daß man mit nur geringem Kaliumfluorid-Überschuß bei entsprechend langer Reaktionszeit immer noch gute Ergebnisse erzielt.

## Patentansprüche

1. Verfahren zur Herstellung von Gemischen, die 2,4,5-Trifluor- und 2-Chlor-4,5-difluor-benzoylfluorid und weniger als 1 Gew.-% 4-Chlor-2,5-difluorbenzoylfluorid enthalten, dadurch gekennzeichnet, daß man 2,4-Dichlor-5-fluor-benzoylchlorid mit der 3,1- bis 4,5-fachen molaren Menge Kaliumfluorid in einem aprotischen Lösungsmittel umsetzt und das Reaktionsgemisch anschließend destilliert.

2. Verfahren nach Ansprüchen 1, dadurch gekennzeichnet, daß man als aprotisches Lösungsmittel Dimethylformamid, Dimethylsulfoxid, N-Methylpyrrolidon, Dimethylsulfon oder Tetramethylensulfon einsetzt.

3. Verfahren nach Ansprüchen 1 bis 2, dadurch gekennzeichnet, daß man es bei Temperaturen im Bereich 150 bis 270°C durchführt.

4. Verfahren nach Ansprüchen 1 bis 3, dadurch gekennzeichnet, daß die Reaktionszeit zwischen 1 und 12 Stunden beträgt.

5. Verfahren nach Ansprüchen 1 bis 4, dadurch gekennzeichnet, daß man es bei einem Druck im Bereich von 0,5 bis 5 bar durchführt.

## Claims

1. Process for the preparation of mixtures containing 2,4,5-trifluoro- and 2-chloro4,5-difluoro-benzoyl fluoride and less than 1% by weight of 4-chloro-2,5-difluoro-benzoyl fluoride, characterized in that 2,4-dichloro-5-fluorobenzoyl chloride is reacted with from 3.1 to 4.5 times the molar amount of potassium fluoride in an aprotic solvent and the reaction mixture is then distilled.

2. Process according to Claim 1, characterized in that the aprotic solvent employed is dimethylformamide, dimethyl sulphoxide, N-methylpyrrolidone, dimethyl sulphone or tetramethylene sulphone.

3. Process according to Claims 1 to 2, characterized in that it is carried out at temperatures in the range from 150 to 270EC.

4. Process according to Claims 1 to 3, characterized in that the reaction time is between 1 and 12 hours.

5. Process according to Claims 1 to 4, characterized in that it is carried out at a pressure in the range from 0.5 to 5 bar.

## Revendications

1. Procédé de préparation de mélanges qui contiennent les fluorures de 2,4,5-trifluoro- et de 2-chloro-4,5-difluorobenzoyle et moins de 1% en poids du fluorure de 4-chloro-2,5-difluorobenzoyle, caractérisé en ce que l'on fait réagir le chlorure de 2,4-dichloro-5-fluorobenzoyle avec une quantité de 3,1 à 4,5 fois en moles de fluorure de potassium dans un solvant aprotique et que l'on distille ensuite le mélange réactionnel.

2. Procédé selon la revendication 1, caractérisé en ce que l'on utilise comme solvant aprotique, le diméthylformamide, le diméthylsulfoxyde, la N-méthylpyrrolidone, la diméthylsulfone ou la tétraméthylènesulfone.

3. Procédé selon les revendications 1 et 2, caractérisé en ce que l'on travaille à une température comprise dans l'intervalle allant de 150 à 270°C.

4. Procédé selon les revendications 1 à 3, caractérisé en ce que la durée de réaction est comprise dans l'intervalle allant de 1 à 12 heures.

5. Procédé selon les revendications 1 à 4, caractérisé en ce que l'on travaille à une pression comprise dans l'intervalle allant de 0,5 à 5 bar.
